(19) Europäisches Patentamt
European Patent Office
Office européen des brevets

(11) **EP 4 174 794 A1**

(12) **EUROPEAN PATENT APPLICATION**

(43) Date of publication:
**03.05.2023 Bulletin 2023/18**

(21) Application number: **21206080.0**

(22) Date of filing: **02.11.2021**

(51) International Patent Classification (IPC):
**G06V 10/82** (2022.01)   **G06V 10/774** (2022.01)
**G06V 10/70** (2022.01)   G01N 33/558 (2006.01)

(52) Cooperative Patent Classification (CPC):
**G06V 10/774; G06V 10/70; G06V 10/82;**
G01N 33/54388

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sensyne Health Group Limited**
**Oxford OX4 4GE (GB)**

(72) Inventors:
• **JAVER GODINEZ, Avelino**
**Oxford, OX4 4GE (GB)**

• **CHUDZIK, Piotr**
**Oxford, OX4 4GE (GB)**
• **YOUSEFI, Paria**
**Oxford, OX4 4GE (GB)**
• **IRVING, Benjamin**
**Oxford, OX4 4GE (GB)**

(74) Representative: **Korenberg, Alexander Tal et al**
**Kilburn & Strode LLP**
**Lacon London**
**84 Theobalds Road**
**London WC1X 8NL (GB)**

(54) **ANOMALY DETECTION IN IMAGES**

(57) A computer-implemented method for detecting an anomalous image using an artificial neural network is provided. A test image is provided as an input to the artificial neural network which was trained on a set of training images to produce images reproducing respective training images input into the artificial neural network in order to produce a reconstructed image. A plurality of regions of the reconstructed image are compared to corresponding regions of the test image to determine a local reconstruction error for each of the plurality of regions. The method further comprises generating an anomaly score from the local reproduction errors. The test image is identified as anomalous with respect to the training set of images based on the anomaly score. Advantageously, the comparison of local variation rather than assessing the read area on a global level enables accurate characterization of local variation even in images that are generally consistent in appearance. Images that are largely untampered with apart from a localised change and therefore have very little global variation are accurately characterized as anomalous using the described method.

200

202 receive and pre-process input data

204 provide pre-processed data as input to trained autoencoder

206 receive reconstruction of pre-processed data as output of trained autoencoder

208 determine local reconstruction errors

210 generate an anomaly score

212 determine whether test is valid using anomaly score

214 notifying user of result

FIG. 2

EP 4 174 794 A1

**Description**

TECHNICAL FIELD

The present disclosure relates to a machine learning system and method for detecting an anomalous image using an artificial neural network, in particular although not exclusively for determining whether an image of a lateral flow tests is anomalous to detect invalid lateral flow tests.

BACKGROUND

Detecting deviations from normality in lateral flow test images is fundamental to accurate diagnosis and to ensure the correct actions are taken in response to a lateral flow test result. One method of detecting abnormalities is by training a supervised classifier to distinguish between normal and abnormal classes. The problem with this approach is that it requires collection of a large number of abnormal samples that capture all the possible variation that will be encountered at inference time. This supervised approach might be sufficient in situations where abnormality is common and well defined. However, in a large population of tests there will always be unexpected and uncommon anomalies that would be unlikely to be detectable using supervised learning techniques.

A lateral flow test has a particular set of challenges with anomaly and fraud not found in other anomaly problems. This includes the possibility that the user will take a photo of another object rather than a lateral flow test, the test itself may have damage to the read area that makes a read undesirable, or the user may have attempted to alter the result of the test with a pen or pencil. The consequence of this is that lateral flow tests may have local errors or variations that affects the read by a deep learning algorithm but does not appear as an anomaly when considering the whole image. For example, a painted line on a test is likely to be ignored if the rest of image is normal, since because the anomaly occupies a small region of the total image, it will be smoothed out unless we can focus on it. This may have unknown consequences for the artificial neural network at inference time as these anomalies may not be found in the original training set.

Unsupervised algorithms may be used to overcome the problems surrounding the lack of abnormal training data. A key challenge of current unsupervised algorithms, such as the GANomaly algorithm (S. Akcay, A. Atapour-Abarghouei, T. P. Breckon, (2018), arXiv:1805.06725v3.), is the need to distinguish different kinds of anomalies, those that indicate an invalid test and those that do not. The algorithm needs to distinguish between normal variation (variation that can still be read correctly by the reader) and abnormal variation (variation that affects the LFT read). Normal variation could include shadows, lighting or images taken with different cameras, and variation in the control and test lines. Abnormal variation could include damaged or tampered tests. Current known unsupervised methods are not effective for identifying relevant variation in images that are generally consistent in appearance (i.e. lateral flow tests). Thus, improved methods detecting an anomalous image, without existing examples of anomalous images are needed, in particular in the context of lateral flow tests but also more widely in any other application domain where it is of interest to detect anomalous images.

SUMMARY

The inventors have realised that detecting abnormal variation within a lateral flow test can be improved by the application of anomaly-based algorithms to the lateral flow read area in order to identify local errors within the image rather than the known method of classing the entire read area as an anomaly. This makes the method particularly effective for lateral flow tests where the images are generally consistent in appearance.

In a first aspect, a method of detecting an anomalous image using an artificial neural network is provided. A test image is provided as an input to the artificial neural network which was trained on a set of training images to produce images reproducing respective training images input into the artificial neural network in order to produce a reconstructed image. A plurality of regions of the reconstructed image are compared to corresponding regions of the test image to determine a local reconstruction error for each of the plurality of regions. The method further comprises generating an anomaly score from the local reproduction errors. The test image is identified as anomalous with respect to the training set of images based on the anomaly score.

Advantageously, the comparison of local variation rather than assessing the read area on a global level enables accurate characterization of local variation even in images that are generally consistent in appearance. Images that are largely untampered with apart from a localised change and therefore have very little global variation are accurately characterized as anomalous using the described method.

The training and test images may comprise respective images of a lateral flow area of a lateral flow antigen test (LFT). The regions may be spaced along the lateral flow read area. The training images may comprise only of valid LFT images, where a valid LFT image describes an image that does not deviate from the normality, where normality is defined by the distribution of data seen in the training set. The reconstruction loss of a normal/valid LFT image does not deviate by more than a predetermined amount from the mean reconstruction loss of the valid LFT training images. The predetermined amount may be three standard deviations from the mean of the distribution of the reconstruction losses, for example. The distribution of the reconstruction losses may be a distribution of the log of the reconstruction losses. An invalid LFT image may deviate by more than, for example, three standard deviations from the distribution seen in the training set. Deviations of normality include random images other than LFTs, damaged devices, devices tampered with, and digital alterations. The anomaly score may be used to determine the validity of the LFT. The generating of the anomaly score may comprise determining the largest one of the local reconstruction errors. The training and test images may be cropped within a bounding box, for example a rectangular bound box comprising the lateral flow read area.

The trained network is only capable of accurately reconstructing similar valid LFT images. If, after training, an input image is an image that deviates from normality, the trained network will produce an output image which is different from the input image and not an accurate reconstruction. LFT images are particular in that in order to falsify a LFT result, only a very small proportion of the image needs to be changed. Likewise, significant damage of the LFT in the image may only cause local deviation in a small section of the image.

Advantageously, the comparison of local variation rather than the scoring the anomaly of the read area on a global level, allows for normal variation such shadows, lighting or images taken with different cameras, and variation in the control and test lines whilst remaining sensitive to abnormal variation that could include damaged or tampered tests.

Each of the plurality of regions may overlap with adjacent regions. The regions may be partially or entirely overlapping with its adjacent regions. For example, the regions may overlap by half on each side of a bounding box strip such each region entirely overlaps its adjacent regions. This ensures that local reconstruction errors that would fit within one region are not overlooked only due to being split between regions.

The artificial neural network may be an autoencoder or convolutional autoencoder_comprising an input layer, an embedding layer and an output layer. The autoencoder network may comprise one or more non-linear intermediate layers between the input and embedding layer, one or more further non-linear intermediate layers between the embedding and output layer and respective network weights for each layer, wherein the dimensionality of the embedding layer is less than that of the input layer._The autoencoder network is trained to reconstruct training images by taking an input image, reducing it into an embedding with a smaller dimensionality than the original input and then reconstructing the embedding to reproduce the original image.

In a second aspect, a method of training an artificial network is provided. The method comprises augmenting the set of training images by adding multiple copies of the same image of a lateral flow test to the set, each copy having a different bounding box. The training images may be augmented by any common technique, for example, the training images may be augmented by random resizing and jittering in order to mimic the uncertainty produced when detecting the bounding box in inference. Advantageously, the augmentation of training data may reduce the model overfitting and make the error distributions between the training and testing more similar.

In a third aspect, a method of training an artificial network is provided. The method comprises receiving an image of a LFT from a user as a test image. The method further comprises identifying if the test image is anomalous and if the test image is identified as anomalous, sending a notification to the user. The notification may be any known method of notifying a user. The notification may be through the same device used by the user to submit the initial test image. Alternatively, the notification may be to an alternative elected device owned by the user or to a device owned by a doctor or employer if appropriate. In some cases, the notification may comprise an SMS, email or application notification. It is understood that any other appropriate notification could be used.

Aspects extend to one or more computer-readable media encoding computer instruction that, when executed on a computing device, implement methods as described above and to a system comprising the one or more computer readable media, a memory for storing the artificial neural network and the data units and a processor for executing the instructions.

The present disclosure refers to artificial neural networks. It will be appreciated that artificial neural networks represent a specific parametrization of a non-linear function (in terms of network weights) and that the present disclosure is not limited by the language used to describe the non-linear function or its structure. It will be understood that an artificial neural network in the context of a computer implemented invention refers to a physical entity that exists either in terms of a physical state of a general purpose or specifically adapted computing platform or in a specifically adapted physical circuitry, for example.

BRIEF DESCRIPTION OF THE DRAWINGS

Illustrative implementations of the present disclosure will now be described, by way of example only, with reference to the drawings. In the drawings:

Figure 1 shows an example of a bounding box and region on a lateral flow test image.

3

**Figure 2** schematically shows a process of detecting an anomalous image;

**Figure 3** shows a diagram of one implementation of the interaction between a user computing device and a second computing device.

**Figure 4** shows a logical block diagram of an autoencoder network 400 for implementing the process;

**Figure 5** shows a logical block diagram of a network for training the autoencoder network;

**Figure 6** schematically shows the process of training an autoencoder suitable for the process of detecting an anomalous image.

**Figure 7** shows the difference in model performance using the entire image vs using patches in inference.

**Figure 8** shows a breakdown of data used for training and testing the model;

**Figure 9** illustrates a block diagram of one implementation of a computing device.

DETAILED DESCRIPTION

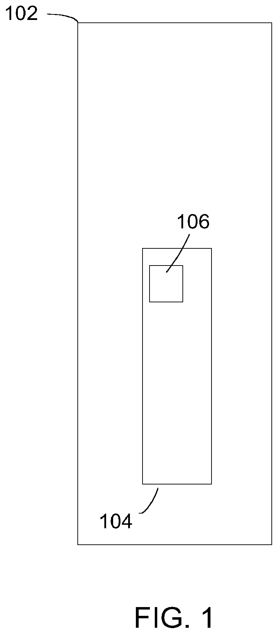

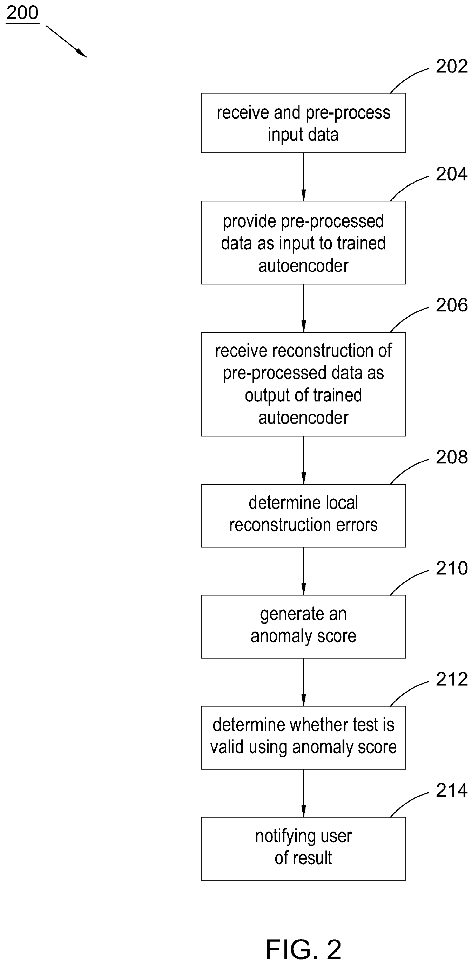

Figure 1 schematically depicts a lateral flow test device image 102 with an example of a bounding box 104 and region 106, as described below with reference to figure 2. It is understood that bounding box 104 and region 106 may be determined for an image of any diagnostic test to be validated, or indeed any image where it is desirable for anomalies to be detected. Figure 2 schematically depicts a process 200 of detecting an anomalous image. Process 200 can be implemented by any kind of general computer or computing device and is specifically suited for computers with a processing capacity adapted for machine learning processes. The process comprises a step 202 of receiving and pre-processing an image. In the described implementation, the image comprises an image of lateral flow test. It will be appreciated that images of different kinds may be used with the described process to implement respective anomaly detection. For example, images of other types of diagnostic tests to be validated. Once received, the image may be pre-processed. Pre-processing may comprise determining the bounding box 104 for each image. In the described implementation, the bounding box 104 is a rectangular box encompassing the lateral flow test result area. The bounding box 104 may be determined using an artificial neural network, ANN. The bounding box 104 may also be an ellipse or any other appropriate shape and may be determined in any suitable way, for example manually or using other image processing techniques. The bounding box 104 identifies a region around the image of the lateral flow test area through which the sample flows. The dependence on the reconstruction error with respect to the dynamic range of the input image (how much contrast there is in the image) may be mitigated by normalizing the image by dividing each pixel value by the pixel range (the maximum minus the minimum pixel value. Intensity normalisation of the image in pre-processing ensures the reconstruction error is independent of the image intensity, therefore enhancing the capacity of the model to deal with low intensity images correctly. The image may then be cropped using the bounding box 104 and resized to a fixed size, for example 128 x 32 pixels.

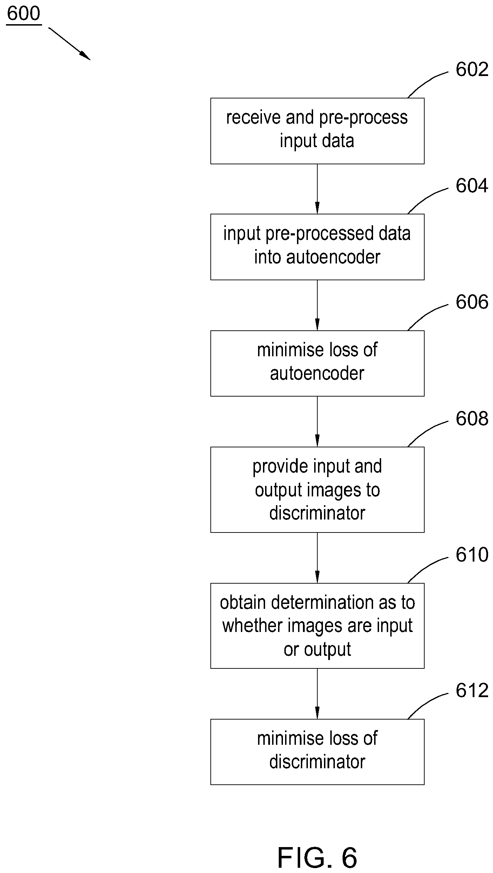

In step 204, the resized image comprising the bounding box region 104 is provided as an input to an autoencoder. The autoencoder has been trained to provide a reconstruction of an image of a lateral flow test according to a process described below with reference to Figure 6. An autoencoder neural network is used in the described implementation but it will be understood that any appropriate neural network trained to reproduce a reconstruction of an image comprising a lateral flow test may be used.

In step 206, a reconstructed image is received as an output to the autoencoder network. In step 208, the reconstructed image is divided into regions, referred to as patches from hereon in and the error for each patch 106 is determined in accordance with equation 1.

$$Patch\ error = \frac{1}{n}\Sigma_i\left(P_i - \hat{P}_i\ \right)^2 \qquad\qquad (1)$$

where $P_i$ and $\hat{P}_i$ correspond to pixel i of n pixels in a given patch of the image X in the input, X and output, X respectively.

In the described implementation the resized 128 x 32 pixel image is divided into 7 overlapping 32 x 32 pixel patches. The patches may for example overlap by half, such that the first patch stretches from pixel 0 to pixel 32 along the second dimension of the resized image, the second patch stretches from pixel 16 to pixel 48, the third patch stretches from pixel 32 to pixel 64 and so on. It will be understood that alternative appropriate patch sizes could be chosen and that likewise alternative overlap of patches could be chosen. For each patch 106, a mean square error is calculated by summing the squared error between two corresponding pixels in the input and output images over all pixels in that patch 106. The patch 106 with the largest error is identified and the corresponding patch error is used as the reconstruction error for that image. In alternative implementations, instead of using the largest patch error as the reconstruction error, the reconstruction error can be based on the patch error in any suitable way, for example using mean or median statistics.

In step 210 an anomaly score is determined for the image in accordance with equation 2. The anomaly score is determined using the highest patch error as described above and therefore represents the reconstruction patch most different from input image X.

$$Anomaly\ Score = \log_{10}(Reconstruction\ error) - Threshold + 1 \qquad (2)$$

The threshold value used to determine the Anomaly Score is obtained from the training set and calculated, using known estimators, for example robust estimators. The use of robust estimators provides estimators more resilient to outliers, for example the use of the median or the top 99 percentile of a distribution instead of the mean. It will be understood that described implementations are not limited to a particular anomaly score, which may, for example, by the reconstruction error or logarithm of the reconstruction error itself, compared against a suitably adjusted threshold, or any other suitable function, including the identity, of the reconstruction error.

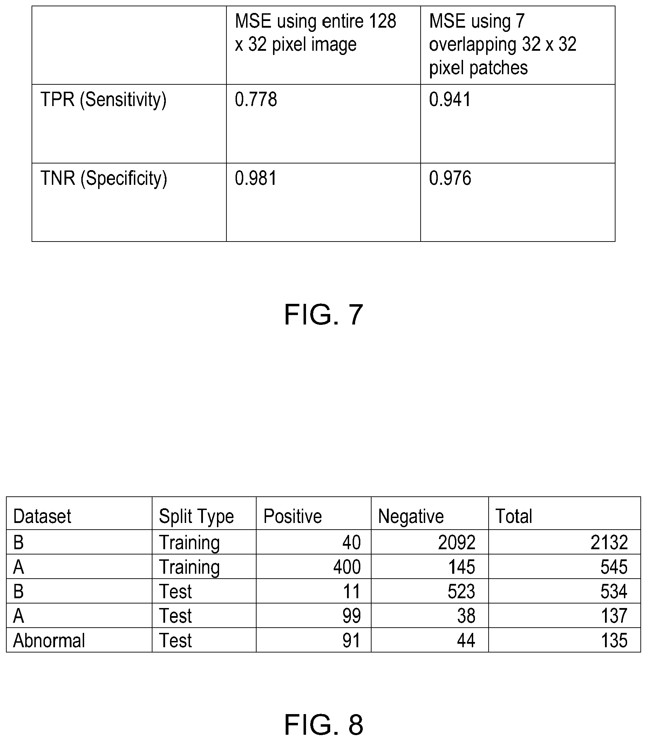

In the described implementation, the threshold is set as three median absolute deviations from the median of the log transform of the distribution of the reconstruction errors from the training set. Other estimators / thresholds, such as multiple of the standard deviation from the mean of the data set or any other suitable function of a measure of variability of the data, may be used. The training set comprises only valid (non anomalous images) and it is assumed that the error distribution for the training and validation sets is similar to the error distribution produced at inference time, hence the threshold is obtained using the training set. Alternatively, if the test and training error distributions are vastly different and re-training is not possible, the threshold may be adjusted by using the same procedure as described above but on a subset of the test set, in cases where the majority of the images in the test dataset are valid images (as described below with reference to figure 8).

An image may be flagged as anomalous if the log reconstruction error of the patch is greater than three median absolute deviations from the median log reconstruction error of the training data. The process 200 is effective to produce an anomaly detector since training the network on valid images makes it unable to accurately reconstruct anomalous images.

In step 212 the validity of the lateral flow test (or other class of object, as the case may be) in the image is determined using the anomaly score. In the described implementation, where the anomaly score is obtained using equation 2, images with an anomaly score larger than 1.0 are considered anomalous.

In optional step 214, the user may be notified of the validity or invalidity of the lateral flow test image. For example, the user may be prompted to take another photo if the original image is rejected by the algorithm as being anomalous/invalid.

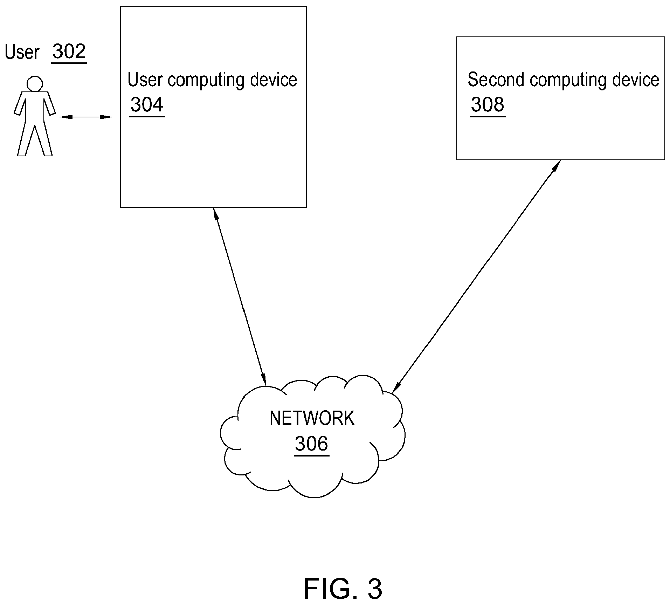

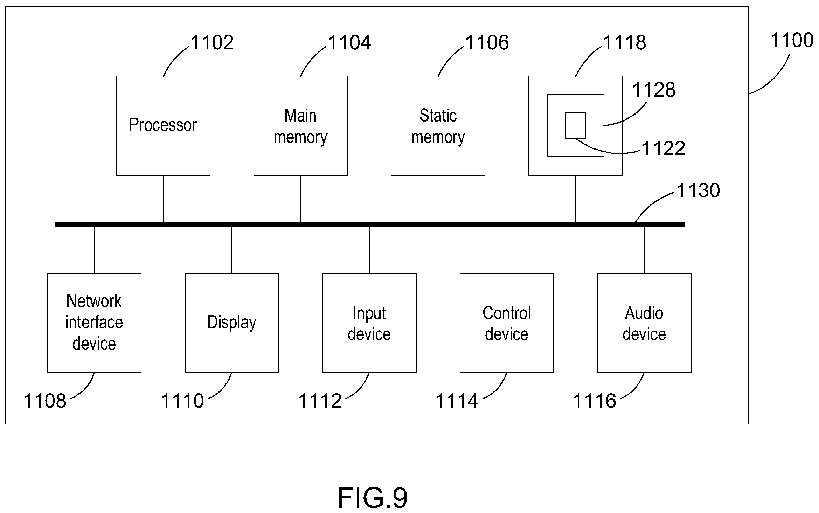

Figure 3 shows a diagram of one implementation of the interaction between a user computing device 304 and a second computing device 308 on which coded instructions to implement process 200 are run. As described in more detail with reference to figure 9, a computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The user computing device 304 may be any device capable of taking and distributing an image, such as a cellular telephone. The image of the LTF is sent from the user device 304 to the second computing device 308 via a network 306 by any appropriate means. For example, the image may be uploaded to a website, sent by MMS or uploaded to a mobile application. Any additional images required from the user 302 due to an anomalous first image or due to an additional LFT being required, may of course be provided to the second computing device 308 as described with reference to the first image. The second computing device 308 may notify the user of the result of the determination as to whether the image of the LTF is valid via the network 306. Additionally, the second computing device 308 may request one or more additional images of the LTF due to invalidity of the LFT. The user 302 may be notified of this request by any appropriate means, such as by SMS, email or mobile application notification from the second computing device 308 to the user computing device 304 via the network 306.

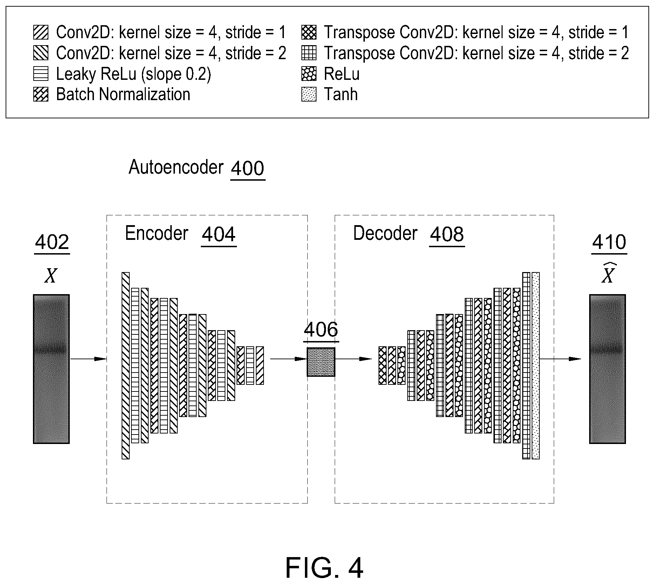

Figure 4 shows a logical block diagram of an autoencoder network 400 for implementing the process 200. The autoencoder network 400 comprises an encoder 404, which generates a low dimensional embedding 406 of the (where applicable pre-processed) input image X 402 and a decoder 408, which takes as an input the low-dimensional embedding 406 and decodes the information in the embedding to provide a reconstruction X 410 of the input image X 402.

The encoder 404 comprises convolutional layers followed by batch normalization layers and layers of leaky rectified linear units (leaky ReLU). The network architecture of a specific implementation is summarised in table 1, below. Features_in and features_out correspond to the number of channels in and channels out which increase as the dimensions of the input image are convoluted The terms channel, kernel, stride and padding are as understood by the skilled person (A. Burkov (2019) The Hundred-Page Machine Learning Book)

*Table 1: Structure of layers of encoder of autoencoder subnetwork 200*

| Encoder |
| --- |
| Conv2d: features_in=3, features_out=64, kernel_size=4, stride=2, padding=1 |
| LeakyReLU: negative_slope=0.2 |
| Conv2d: features_in=64, features_out=128, kernel_size=4, stride=2, padding=1 |
| BatchNorm2d |
| LeakyReLU: negative_slope=0.2 |
| Conv2d: features_in=128, features_out=256, kernel_size=4, stride=2, padding=1 |
| BatchNorm2d |
| LeakyReLU: negative_slope=0.2 |
| Conv2d: features_in=256, features_out=512, kernel_size=4, stride=2, padding=1 |
| BatchNorm2d |
| LeakyReLU: negative_slope=0.2 |
| Conv2d: features_in=512, features_out=1 024, kernel_size=4, stride=2, padding=1 |
| BatchNorm2d |
| LeakyReLU: negative_slope=0.2 |
| Conv2d: features_in=1024, features_out=50, kernel_size=4, stride=1, padding=0 |

In the specific implementation summarised by table 1, the 2D convolutional layers (Conv2d) have kernel size 4 and stride 2, with the exception of the final layer which has kernel size 4 and stride 1. The first convolutional layer receives 3 channels; red, green and blue, as typical for a colour image, and outputs 64 channels by applying 64 kernels of size 4 to the input. The use of strided convolutions removes the requirement for pooling layers by decreasing the dimension by jumping multiple pixels between convolutions. In the specific implementation summarised by table 1, stride 2 convolutions are used which half the height and width of the input to each convolutional layer. Leaky ReLU are applied after each convolutional layer, with the exception of the final convolutional layer, in order to introduce nonlinearity. Leaky ReLU have a small predetermined slope when x < 0. In the described implementation, y = 0.2x when x < 0. It should be understood that alternative nonlinear functions like the tanh and sigmoid functions may also be used. Batch normalization (BatchNorm2d) is used to normalise the inputs to the batch normalisation layer within the network to have zero mean and unit variance at the output of the batch normalisation layer.

The decoder uses layers which perform transformations in the opposite direction of a normal convolution (ConvTransponse2d), batch normalization layers and ReLU with the exception of the final layer which uses tanh activation in accordance with table 2, below. The tanh activation layers forces the output to be bounded between -1 and -1, in the same range as the intensity normalized input, which is bounded between 1 and -1, ConvTranspose2d layers, also referred to as transpose convolutional layers or inverse convolutional layers are widely known and used. For the sake of brevity, they are only briefly described here. ConvTranspose2d layers perform transformations in the opposite direction of a normal convolution i.e. from something that has the shape of the output of some convolution to something that has the shape of its input, while maintaining a connectivity pattern that is compatible with said convolution. In the described implementation, ConvTranspose2d layers are used to learn a set of weights that can be used to reconstruct the high-resolution image X as X from the low dimensional embedding 406. The transpose convolutional layers have kernel size 4 and stride 2, with the exception of the first layer which has kernel size 4 and stride 1.

*Table 2: Structure of layers of decoder of autoencoder subnetwork 200*

| Decoder |
| --- |
| ConvTranspose2d: features_in=50, features_out=1024, kernel_size=4, stride=1, , padding=0 |
| BatchNorm2d |
| ReLU |
| ConvTranspose2d: features_in=1024, features_out=512, kernel_size=4, stride=2, padding=1 |

(continued)

| Decoder |
| --- |
| BatchNorm2d |
| ReLU |
| ConvTranspose2d: features_in=512, features_out=256, kernel_size=4, stride=2, padding=1 |
| BatchNorm2d |
| ReLU |
| ConvTranspose2d: features_in=256, features_out=128, kernel_size=4, stride=2, padding=1 |
| BatchNorm2d |
| ReLU |
| ConvTranspose2d: features_in=128, features_out=64, kernel_size=4, stride=2, padding=1 |
| BatchNorm2d |
| ReLU |
| ConvTranspose2d: features_in=64, features_out=3, kernel_size=4, stride=2, padding=1 |
| Tanh |

Other suitable implementations trained to convert an input image into a reconstruction of the input image are of course also possible, including other number of layers, architectures and activations.

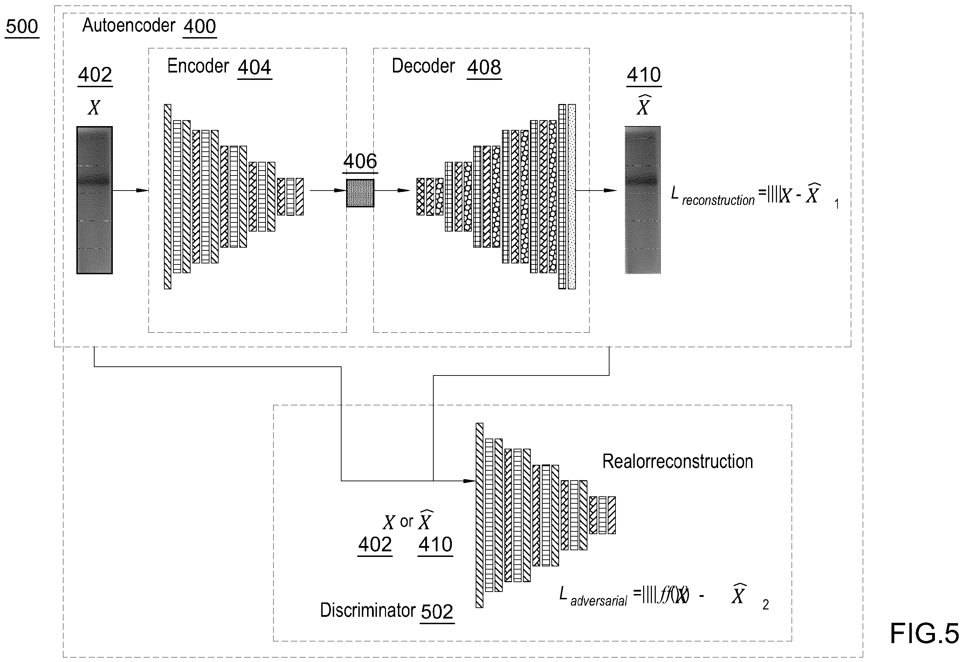

Figure 5 shows a logical block diagram of a system for training the autoencoder network 400. The system 500 optionally comprises a discriminator network 502 in addition to the autoencoder network 400. In the described implementation, the discriminator network 502 is used to determine one or more additional losses that are used in training the autoencoder network 400 as described below. It is however understood, that the autoencoder network 400 may be trained without the discriminator network.

As described above with reference to figure 4, the autoencoder network 400 takes input data, X and outputs a reconstruction, X. The discriminator network 502 takes as input X and X and outputs a prediction of whether each image is either an input image X or a reconstruction X.

Figure 6 schematically depicts a process 600 of training the autoencoder 400 to learn an embedding suitable for performing process 200. The process comprises a step 602 of receiving and pre-processing an image. In the described implementation, the image comprises an image of lateral flow test. It is understood that autoencoder network 400 may be trained to detect anomalies in other images, for example images of other types of diagnostic tests to be validated, or indeed of any other type of object to be validated, that is to be compared to a set or distribution of valid objects.

As described with reference to the step 402, step 602 comprises determining a bounding box for each image. In addition, the image may be altered using common augmentations like image rotation (-5° to 5° taken from a uniform distribution) and flipping. Random jittering may be introduced, (randomly between -10% and 10% of the width for the horizontal coordinates, and of the height in the vertical coordinates) and the bounding box randomly resized (randomly between 80% and 125% of the original width and height independently). The bounding box may be augmented by random resizing and jittering in order to mimic the uncertainty produced when detecting the bounding box in inference. The augmentation of training data may reduce the model overfitting and make the error distributions between the training and validation datasets more similar.

The image is cropped using the bounding box and resized to a fixed size (32 x 128) before being provided as an input image, X 402 to the autoencoder network 400 at step 604.

The autoencoder network 400 learns to reconstruct image X, 402 as X, 410 from the low dimensional embedding 406 by minimizing the difference between the image X and the reconstruction X using an L1 reconstruction loss, described in equation 3:

$$\text{Reconstruction loss} = || X - \hat{X} ||_1 \qquad (3)$$

where $\| \cdot \|_1$ denotes the mean of the absolute values also known as absolute loss.

At step 608, the discriminator sub-network 502 takes as input X and X and outputs a prediction for each input image of whether each image was X or X in step 410. The autoencoder network 400 may additionally use an adversarial loss, obtained from the discriminator network, in accordance with equation (4).

$$\text{Adversarial loss} = ||f(X) - f(\hat{X})||_2 \qquad (4)$$

where f is a function that outputs an intermediate layer of the discriminator, f is applied to the feature representations of original input X and reconstruction X, such that the difference between the feature representations are minimised when the loss is backpropagated through the autoencoder network 400 and $||\cdot||_2$ denotes the mean of the squared values to give a least square loss. It is understood that the use of adversarial loss to train the autoencoder network 400 is optional and in alternative implementations, the autoencoder network 400 is trained using only the reconstruction loss or any appropriate loss to minimise the difference between input image X, 402 and reconstruction X, 410. Furthermore, although the absolute loss is used to determine the reconstruction loss and the least square loss is used to determine the adversarial loss, it is understood that any combination of loss functions may be used to appropriately determine reconstruction and adversarial loss.

In the described implementation, the weights of the autoencoder network 400 are updated using a combination of the Adversarial and the Reconstruction loss whilst the weights of discriminator network 502 are kept constant. The training process 600 iterates between adjusting the weights of autoencoder network 400 in step 606 and the discriminator network in step 612. In the described implementation, autoencoder network 400 is trained over 1000 iterations, however it is understood that any appropriate number of iterations or alternatives such as the use of a threshold loss could also be used. Furthermore, checkpoints may be saved during training and the checkpoint with the lowest Reconstruction error when performing on the test data, isolated and used for inference. During the discriminator training, the autoencoder network 400 does not train, its weights remain constant as it produces samples of pairs of input, X and reconstructed images, X for the discriminator to train on. The discriminator loss function penalises the discriminator for misclassifying the images.

The discriminator network 502 uses binary cross entropy, BCE, in accordance with equation 5, to learn to classify its input image as either an input image X or reconstructed image X.

$$\text{BCE} = -\Sigma_j t_j \log(p_j) \qquad (5)$$

where $t_j$ is the ground truth of the discrete variable, in the described implementation and $p_j$ is the probability of discrete variable 'j' in the prediction.

In the optimization of the discriminator, noisy labels are used, such that instead of predicting 1 for X and 0 for X, a random number between 0.8 and 0.9 is predicted for the X samples, and a random number between 0.1 and 0.3 is predicted for the X samples.

The system 500 is used to train the autoencoder network 400 to minimize the reconstruction and the adversarial loss and discriminator network 502 to minimize the binary cross entropy loss. In this way, the autoencoder network 400 is trained to create reconstructions capable of fooling the discriminator network 502 and hence penalises the autoencoder network for failing to cause the discriminator to mis-classify the image.

In the described implementation, different learning rates are used for the discriminator network 502 and the autoencoder network 400 in order to mitigate the problem found in the known GANomaly model, where the discriminator network 502 learns disproportionately quickly and constantly resets the weights autoencoder network 400 with random numbers.

As mentioned above with reference to the adversarial loss, in alternative implementations, the autoencoder network 400 is trained using only the reconstruction loss and hence no discriminator network is used.

Figure 7 shows the difference in model performance using the entire image vs using patches in inference. The model was trained as described above with reference to figure 6. The performance of the model was then assessed using two metrics; true positive rate (TPR) and true negative rate (TNR). The true positive rate indicates the proportion of images that are genuinely anomalous that give a anomalous result using the trained model, this indicates the sensitivity of the model. The true negative rate gives the proportion of images that are valid that give a result of valid using the model, this indicates the specificity of the model. The first column of figure 7 indicates the proportion of TPR and TNR when the MSE of the entire image is determined in inference.

The process to determine the MSE of the entire image is similar to the process described with reference to figure 2 with the exception of step 208 "determine local reconstruction losses". In replacement of step 208, the reconstruction error of the entire image is determined for use in step 210, generating an anomaly score. The process to determined the MSE of the patches is as described above with reference to figure 2.

The first row of figure 7 shows sensitivities of 0.778 and 0.941 using the error from the entire image vs the error in the patches respectively. This shows that the use of patches in inference vastly improves the detection of genuine anomalies. The second row of figure 7 shows specificity's of 0.981 and 0.976 using the error from the entire image vs the error in the patches respectively. This that using the patches has a marginally higher proportion of valid images being flagged as anomalous.

Figure 8 shows the breakdown of data used for training and testing the model. The model was trained as described above with reference to figure 6. The training data comprised only valid images of lateral flow tests where testing was carried out according to manufacturer instructions. Valid images may be obtained from test sites where lateral flow testing is performed by a supervised operative as per the manufacturer instructions. Alternatively, valid images of lateral flow tests may be obtained from self-testing users and validated as per the manufacturers instructions for use. The images of the lateral flow test may be taken on a standardised device.

The training data, shown in the first two rows of figure 8, was made up of two datasets A and B, with the testing data, shown in the last three rows, comprising an additional dataset of abnormal images. The A dataset used for training comprised a total of 545 samples with an average image size of 1200 x 900 pixels and bounding boxes obtained using a detection network. The B dataset used for training comprised a total of 2132 samples with an average image size of 4032 x 2250 pixels and manually annotated bounding boxes.

In both A and B training datasets, detecting regions were manually filtered in order to remove abnormalities such as leaks and shadows. The A and B dataset comprised images of both positive and negative test results in order to ensure the trained network could accurately reconstruct both test results in images of lateral flow tests. The A training dataset comprised 400 positive results and 40 negative results and the B training dataset comprised 40 positive results and 2092 negative results as shown in the positive and negative columns of figure 8.

The model performance was tested using the process described above with reference to Figure 2. The testing dataset comprises an additional 137 images from dataset A, 534 images from dataset B and further 137 images from a dataset of abnormal images. As with the training data, the test dataset comprised images of both positive and negative results as shown in figure 8.

The Abnormal dataset was used only for testing the model so as not to train the model on abnormal images. The images in the abnormal dataset were created by digitally modifying images from dataset B, for example by the addition or alteration of lines on the lateral flow test read area or by removing sections of the read area from the image. Alternation of the images was performed in order to mimic images where the results are falsified or the LTF in the image is damaged.

Example Hardware Implementation

Figure 9 illustrates a block diagram of one implementation of a computing device 900 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 900 includes a processing device 902, a main memory 904 (e.g., read-only memory (ROM), flash memory, dynamic random access memory (DRAM) such as synchronous DRAM (SDRAM) or Rambus DRAM (RDRAM), etc.), a static memory 906 (e.g., flash memory, static random access memory (SRAM), etc.), and a secondary memory (e.g., a data storage device 918), which communicate with each other via a bus 930.

Processing device 902 represents one or more general-purpose processors such as a microprocessor, central processing unit, or the like. More particularly, the processing device 902 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 902 may also be one or more special-purpose processing devices such as an application specific integrated circuit (ASIC), a field programmable gate array (FPGA), a digital signal processor (DSP), network processor, or the like. Processing device 902 is configured to execute the processing logic (instructions 922) for performing the operations and steps discussed herein.

The computing device 900 may further include a network interface device 908. The computing device 900 also may include a video display unit 910 (e.g., a liquid crystal display (LCD) or a cathode ray tube (CRT)), an alphanumeric input device 912 (e.g., a keyboard or touchscreen), a cursor control device 914 (e.g., a mouse or touchscreen), and an audio device 916 (e.g., a speaker).

The data storage device 918 may include one or more machine-readable storage media (or more specifically one or more non-transitory computer-readable storage media) 928 on which is stored one or more sets of instructions 922 embodying any one or more of the methodologies or functions described herein. The instructions 922 may also reside, completely or at least partially, within the main memory 904 and/or within the processing device 902 during execution thereof by the computer system 900, the main memory 904 and the processing device 902 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining," "identifying", "providing", "applying", "training" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A method of detecting an anomalous image, the method comprising

   inputting a test image into an artificial neural network, trained on a set of training images to produce images reproducing respective training image input to the artificial neural networks, to produce a reconstructed image;
   comparing a plurality of regions of the reconstructed image to corresponding regions of the test image to determine a local reconstruction error for each of the plurality of regions;
   generating an anomaly score from the local reproduction errors; and
   identifying the test image as anomalous with respect to the training set of images based on the anomaly score.

2. The method of claim 1, wherein the training images and the test image comprise respective images of a lateral flow area of a lateral flow antigen test and the training images are of valid lateral flow antigen tests.

3. The method of claim 2, where in the regions are spaced along the lateral flow read area.

4. The method of claim 2 or 3, wherein the training images and the test image are cropped to within a bounding box, for example a rectangular bounding box, comprising the lateral flow area.

5. The method of claim 2, 3 or 4, comprising using the anomaly score to determine if the lateral flow test is valid.

6. The method of any preceding claim, wherein each region overlaps with an adjacent region.

7. The method of claim 6, wherein each region fully overlaps with its adjacent regions.

8. The method of any preceding claim, wherein the artificial neural network is an autoencoder.

9. The method of claim 8, wherein the artificial neural network is a convolutional autoencoder.

10. The method of any preceding claim, wherein generating the anomaly score comprises determining the largest one of the local reconstruction errors.

11. A method of training an artificial neural network for use in a method according to claim 4 or 5, or any one of claims 6 to 10 when dependent on claim 4, the method comprising augmenting the set of training images by adding multiple copies of the same image of a lateral flow test to the set, each copy having a different bounding box.

12. A method of processing images of lateral flow tests, the method comprising:

   receiving an image of a lateral flow test from a user as a test image;
   identifying if the test image is anomalous using a method according to any one of claims 1 to 10; and
   if the test image is identified as anomalous, sending a notification to the user.

13. The method of claim 12, comprising requesting that the user submits another image of a lateral flow test.

14. One or more computer-readable media comprising coded instructions that, when run on a processor, implement a method according to any preceding claim.

15. A system comprising a computer processor and memory, the memory storing coded instructions that, when run on the processor, implement a method according to any one of claims 1 to 13.

FIG. 1

200

| receive and pre-process input data | 202 |

| provide pre-processed data as input to trained autoencoder | 204 |

| receive reconstruction of pre-processed data as output of trained autoencoder | 206 |

| determine local reconstruction errors | 208 |

| generate an anomaly score | 210 |

| determine whether test is valid using anomaly score | 212 |

| notifying user of result | 214 |

FIG. 2

User  302

User computing device
304

Second computing device
308

NETWORK
306

FIG. 3

Conv2D: kernel size = 4, stride = 1    Transpose Conv2D: kernel size = 4, stride = 1
Conv2D: kernel size = 4, stride = 2    Transpose Conv2D: kernel size = 4, stride = 2
Leaky ReLu (slope 0.2)    ReLu
Batch Normalization    Tanh

Autoencoder 400

402
$X$

Encoder 404

406

Decoder 408

410
$\widehat{X}$

FIG. 4

FIG.5

600

602

receive and pre-process
input data

604

input pre-processed data
into autoencoder

606

minimise loss of
autoencoder

608

provide input and
output images to
discriminator

610

obtain determination as to
whether images are input
or output

612

minimise loss of
discriminator

FIG. 6

|  | MSE using entire 128 x 32 pixel image | MSE using 7 overlapping 32 x 32 pixel patches |
|---|---|---|
| TPR (Sensitivity) | 0.778 | 0.941 |
| TNR (Specificity) | 0.981 | 0.976 |

# FIG. 7

| Dataset | Split Type | Positive | Negative | Total |
|---|---|---|---|---|
| B | Training | 40 | 2092 | 2132 |
| A | Training | 400 | 145 | 545 |
| B | Test | 11 | 523 | 534 |
| A | Test | 99 | 38 | 137 |
| Abnormal | Test | 91 | 44 | 135 |

# FIG. 8

EP 4 174 794 A1

**FIG.9**

Europäisches
Patentamt

European
Patent Office

Office européen
des brevets

# EUROPEAN SEARCH REPORT

Application Number

EP 21 20 6080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/137745 A1 (UNIBAP AB [SE]) 8 July 2021 (2021-07-08) * figures 5-8 * * page 2, column 4 - page 4, column 3 * * page 11, column 21 - page 15, column 8 * | 1-15 | INV. G06V10/82 G06V10/774 G06V10/70 ADD. G01N33/558 |
| X | AKCAY SAMET ET AL: "Skip-GANomaly: Skip Connected and Adversarially Trained Encoder-Decoder Anomaly Detection", 2019 INTERNATIONAL JOINT CONFERENCE ON NEURAL NETWORKS (IJCNN), IEEE, 14 July 2019 (2019-07-14), pages 1-8, XP033621656, DOI: 10.1109/IJCNN.2019.8851808 [retrieved on 2019-09-27] * Section III * | 1-15 | |
| A | MOHAMMAD SABOKROU ET AL: "Adversarially Learned One-Class Classifier for Novelty Detection", ARXIV.ORG, CORNELL UNIVERSITY LIBRARY, 201 OLIN LIBRARY CORNELL UNIVERSITY ITHACA, NY 14853, 25 February 2018 (2018-02-25), XP081232164, * the whole document * | 1-15 | TECHNICAL FIELDS SEARCHED (IPC) G06V |

The present search report has been drawn up for all claims

| Place of search | Date of completion of the search | Examiner |
|---|---|---|
| The Hague | 7 April 2022 | Borges, Pedro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 6080

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2022

| Patent document cited in search report | | Publication date | Patent family member(s) | | Publication date |
|---|---|---|---|---|---|
| WO 2021137745 A1 | | 08-07-2021 | SE | 1930421 A1 | 01-07-2021 |
| | | | WO | 2021137745 A1 | 08-07-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **S. AKCAY ; A. ATAPOUR-ABARGHOUEI ; T. P. BRECKON.** *arXiv:1805.06725v3,* 2018

- **A. BURKOV.** *The Hundred-Page Machine Learning Book,* 2019